# EUROPEAN PATENT SPECIFICATION

(11) **EP 2 088 940 B1**
(45) Date of publication and mention of the grant of the patent: **11.05.2011**
(21) Application number: 07865276.5
(22) Date of filing: 06.12.2007
(51) Int. Cl.: A61B 17/22, A61B 10/04, A61B 17/00, A61B 17/32, A61B 10/02

(54) **WIRE-GUIDED CURETTE**
DRAHTGEFÜHRTE KÜRETTE
CURETTE FILOGUIDÉE

(30) Priority: 08.12.2006 US 873675 P
(43) Date of publication of application: 19.08.2009
(73) Proprietor: Wilson-Cook Medical Inc., Winston-Salem, NC 27105-4191 (US)
(72) Inventor: HARDIN, David M., Winston-Salem, NC 27106 (US)
(74) Representative: Garratt, Peter Douglas
(86) International application number: PCT/US2007/086586
(87) International publication number: WO 2008/073792

(56) References cited:
- EP-A- 0 084 251
- WO-A-2006/058328
- WO-A-2006/100882
- US-A- 4 243 049
- US-A- 4 378 811
- US-A- 4 951 684
- US-A- 5 762 070
- US-A1- 2002 120 283
- US-A1- 2005 043 721
- US-A1- 2005 059 890
- US-B1- 6 607 494

## Description

### TECHNICAL FIELD

The invention generally relates to a medical curette device that can be navigated over a wire guide.

### BACKGROUND

A curette is a medical instrument that is used to remove tissue during a curettage medical procedure. The end of the curette is generally a spoon-like shape that contacts the target tissue to be removed. The target tissue that is removed can thereafter be sampled. The tissue may be used for cytological and/or histological diagnosis.

Conventional curettes include ear, uterine, and dermal curettes. They are hand-held devices that generally do not require significant navigation to reach the target tissue to be sampled.

Conventional curettes are not capable of navigating remote body cavities, such as the gastrointestinal cavity. Additionally, the anatomy of the gastrointestinal cavity contains many tortuous body lumens which make accessing these cavities difficult. Document WO-A-2006/050328 discloses a wire-guided tubular tissue sampling fine needle.

In view of the difficulties to successfully perform curettage within remote body cavities, there is an unmet need for a curette that can reliably gain access to remote body cavities and effectively remove tissue therefrom.

### SUMMARY

Accordingly, a wire-guided curette is provided that addresses one or more of the needs described above, as claimed in claim 1 with preferred embodiments in the dependent claims.

In a first aspect of the invention, a wire-guided curette device is provided comprising a tubular shaft, a scraping element, and a wire guide lumen. The tubular shaft extends between a proximal portion and a distal portion. The distal portion is adapted to be introduced into a body lumen and the proximal portion is adapted to remain outside of the lumen so as to allow manipulation of the distal portion. The scraping element is operably connected to the distal portion of the tubular shaft. The scraping element has at least one surface configured to remove tissue from the body lumen. A wire guide lumen extends longitudinally through at least the distal portion of the tubular shaft. The wire guide lumen comprises an aperture disposed on or near the scraping element. The wire guide lumen is adapted to receive a wire guide.

In a second aspect of the invention, a wire-guided curette device is provided comprising a shaft and a curvilinear segment. The shaft extends between a proximal portion and a distal portion. The distal portion is adapted to be introduced into a body lumen. The proximal portion is adapted to remain outside of the lumen so as to allow manipulation of the distal portion. The shaft comprises a first lumen adapted to receive a wire guide. The curvilinear segment is operably connected to the distal portion. The curvilinear segment is offset from a longitudinal axis of the shaft to allow the wire guide to exit an opening in the distal portion without substantially contacting a surface of the curvilinear segment. The curvilinear segment is configured to remove tissue from a body lumen during a curettage procedure.

The device of invention can be used in a method of performing a curettage procedure. The wire-guided curette device includes a tubular shaft extending between a proximal portion and a distal portion. The distal portion is adapted to be introduced into a body lumen, and the proximal portion adapted to remain outside of the lumen so as to allow manipulation of the distal portion. The device also includes a scraping element which is operably connected to the distal portion of the tubular shaft. The scraping element has at least one surface configured to remove tissue from the body lumen. A wire guide lumen extends longitudinally through at least the distal portion of the tubular shaft. The wire guide lumen comprises an aperture disposed near the scraping element. The wire guide lumen is adapted to receive a wire guide. An endoscope is advanced towards a target tissue site. A wire guide is loaded through an accessory channel of the endoscope to the target tissue site. The wire-guided curette device is advanced over the wire guide towards the target tissue site. The scraping element is engaged with the target tissue site, such that at least one surface of the scraping element contacts the target tissue.

### BRIEF DESCRIPTION OF THE DRAWINGS

Embodiments will now be described by way of example with reference to the accompanying drawings, in which:

Figure 1 is a perspective view of a wire-guided curette;

Figure 2 is side view of the wire-guided curette of Figure 1;

Figure 3 is a perspective view of a wire-guided curette with multiple scraping elements not according to the invention;

Figure 4 is a side view of a distal portion of a wire-guided curette with an angled loop-shaped scraping element not according to the invention;

Figure 5 is a side view of a distal portion of a wire-guided curette with an angled triangular-shaped scraping element not according to the invention;

Figure 6 is a perspective view of the wire guided curette of Figure 1 with a shortened wire guide lumen;

Figure 7 is a perspective view of a protective sheath disposed over the wire-guided curette; and

Figure 8 shows a side view of an endoscope being advanced within a desired region of the gastrointestinal lumen where the target tissue to be removed is located.

### DETAILED DESCRIPTION

The embodiments are described with reference to the drawings in which like elements are referred to by like numerals. The relationship and functioning of the various elements of the embodiments are better understood by the following detailed description. However, the embodiments as described below are by way of example only, and the invention is not limited to the embodiments illustrated in the drawings. It should also be understood that the drawings are not to scale and in certain instances details have been omitted, which are not necessary for an understanding of the embodiments, such as conventional details of fabrication and assembly.

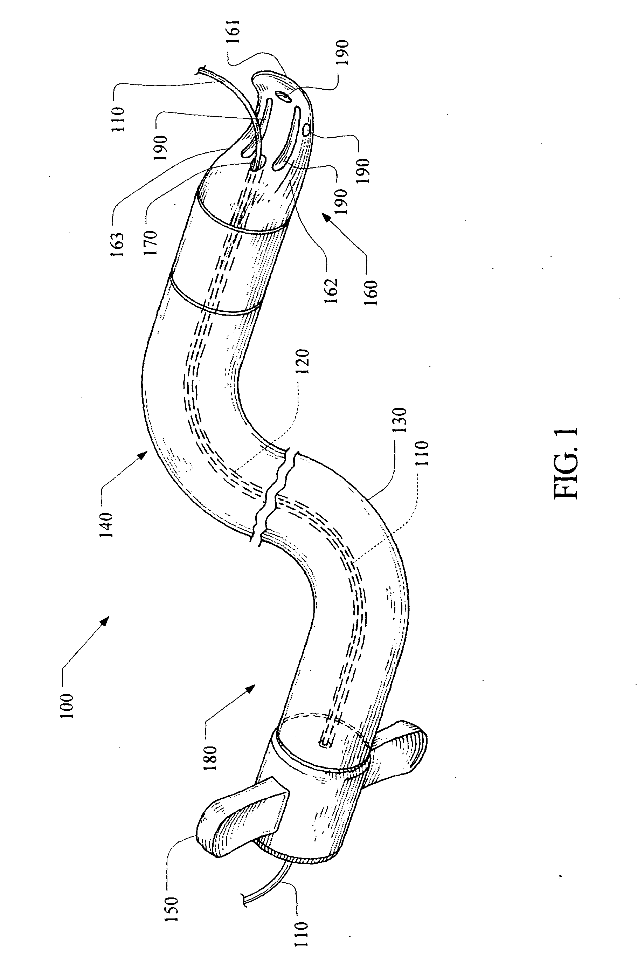

An exemplary wire-guided curette is shown in Figure 1. Figure 1 shows a wire-guided curette 100 that is loaded with a wire guide 110. The wire-guided curette 100 includes a tubular shaft 130 having a distal portion 140 and a proximal portion 180. A scraping element 160 is affixed to the distal portion 140 of the tubular shaft 130. In general, the wire-guided curette 100 can be navigated over the wire guide 110 to gain access to remote body cavities. After reaching the target tissue site, the scraping element 160 can engage the target tissue and remove a desired amount of the tissue for sampling.

Tubular shaft 130 includes a distal portion 140 and a proximal portion 180. The distal portion 140 of the shaft 130 is designed to be introduced into a body lumen of a patient, where the curettage procedure is to be performed. The proximal portion 180 of the shaft 130, which includes the control handle 150, is designed to remain outside of the patient's body so that the distal portion of the 140 of the shaft 130 may be manipulated inside a body lumen by a physician handling the control handle 150.

Tubular shaft 130 may be flexible. Tubular shaft 130 may be formed from any semi-rigid polymer such as polyurethane, polyethylene, polypropylene, tetrafluroethylene, polytetrafluoroethylene, fluorinated ethylene propylene, or the like. Tubular shaft 130 may also be coated with any hydrophilic polymer known to one of ordinary skill in the art to promote wetability of the surface of the shaft 130 as it navigates through the body lumen. The wetability reduces the friction of the shaft 130 during deployment, thereby reducing trauma incurred by the patient during the curette procedure. The reduced friction and flexibility of the shaft 130 enables it to be navigated through the body lumen of the patient, as well as to flex and bend during the curettage procedure, which will be described below. Tabular shaft 130 preferably has a length ranging between about 80 centimeters and about 200 centimeters, and more preferably, between about 100 and about 180 centimeters. The tubular shaft 130 preferably has a diameter ranging between about 3 FR and about 12 FR.

Tubular shaft 130 includes wire guide lumen 120, as shown in Figure 1. Wire guide lumen 120 extends from the distal portion 140 to the proximal portion 180. Wire guide lumen 120 preferably has a diameter between about 0.012" and about 0.070". Wire guide 110 is shown disposed through wire guide lumen 120 and exiting through aperture 170. Although the wire guide lumen 120 is shown to extend to the control handle 150, the wire guide lumen 120 may also extend to a side port located along the proximal portion 180 through which the wire guide may be fed.

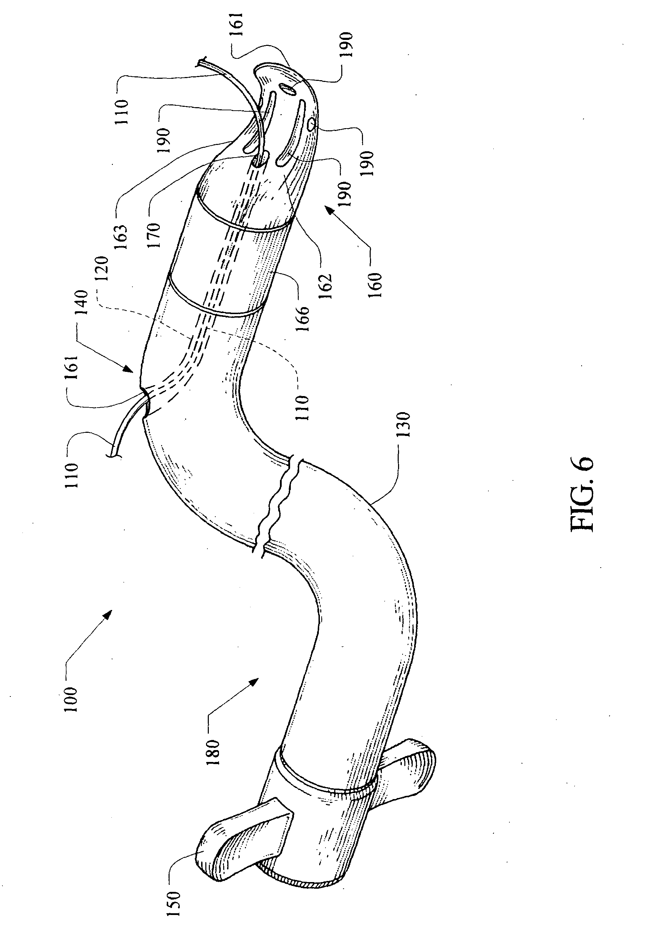

Other variations of the wire guide lumen 120 are contemplated. For example, Figure 6 shows that the curette 100 may contain a wire guide lumen 120 that extends only within the distal portion 140. Figure 6 shows an intermediate wire guide port 181 that the wire guide 110 passes through so as to feed into the wire guide lumen 120. The wire guide lumen 120 extends distally of the port 181 and exits through aperture 170. Only the distal end of the wire guide 100 is within the lumen 120, thereby enabling an intermediate wire guide exchange or release of the wire guide 110 from the curette 100. The proximal portion of the wire guide 110 remains outside of the curette 100. Release of the wire guide 110 from the curette 100 may be achieved by pulling the wire guide 110 proximally until the distal portion of wire guide 110 has been removed from the lumen 120. Alternatively, release of the wire guide 110 from the curette 100 may be achieved by pushing the curette 100 distally until port 181 passes distally beyond the distal end of the wire guide 110. Additional details of these methods, which are referred to as interductal exchanges, are disclosed in U.S. Publication No. 2005-0070794 A1, published on March 31, 2005.

Alternatively, the curette 100 may be separated from the wire guide 100 by withdrawing the curette 100 proximally until it passes over the proximal end of the wire guide 110. Because the devices are not being exchanged over the entire length of the wire guide 110, a short wire guide exchange is possible. Such a short wire guide exchange may decrease surgical procedure time. After separation of the curette 100 from the wire guide 110, other devices may be fed over wire guide 110, which is already inserted at the target site. Alternatively, the wire guide lumen 120 may extend the entire length of the shaft 130 to support both short and long wire guide exchanges.

Although a single wire guide lumen 120 is shown in Figure 1, additional lumens may also be disposed through shaft 130. For example, a second lumen may be employed to inject contrast media. The second lumen may exit through a second aperture spaced away from aperture 170.

Scraping element 160 is shown in Figure 1 to be affixed at the distal portion 140 of the tubular shaft 130. The scraping element 160 is shown to be generally spoon-shaped. Scraping element 160 has a curved inner surface 162, a curved outer surface 161, and an edge 163. Any surface of the scraping element 160 may be used to engage target tissue or strictures. For example, the outer surface 161 may engage the target tissue via back and forth movements to scrape and peel away cells. Edge 163 may also engage the target tissue and be used to apply pressure to cells that are more difficult to extract. Alternatively, a combination of the outer surface 161, inner surface 162, and edge 163 may be used to scrape cells.

Additionally, the spoon-shape geometry facilitates a scoop-like movement that may be used to extract the target tissue for subsequent sampling. The scoop-like movement also assists in sweeping away or excising strictures that may impede passage through the body lumen.

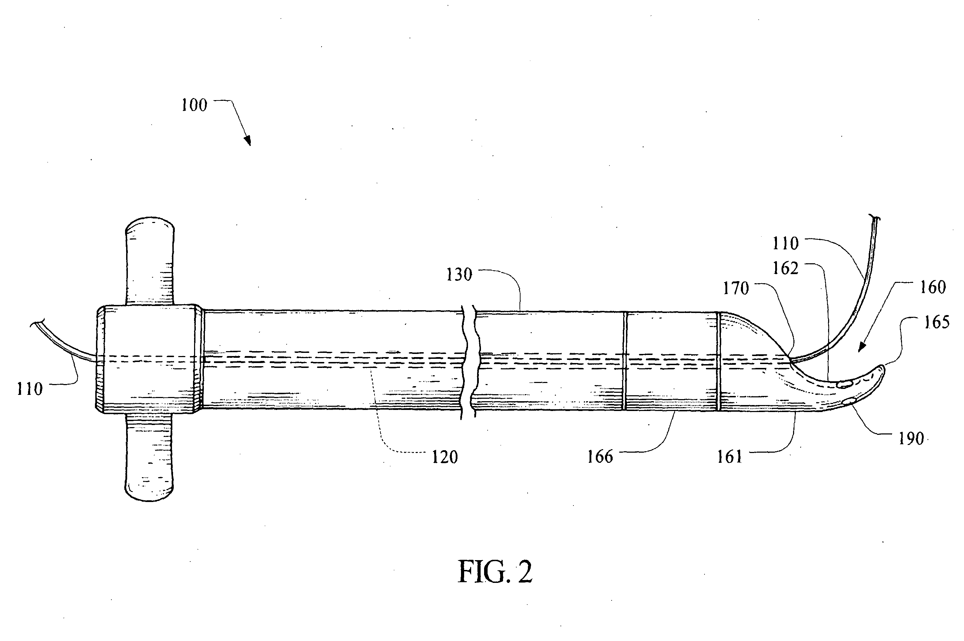

The collection of target tissue may be achieved with reservoirs 190, as shown in Figures 1 and 2. Reservoirs 190 help to trap the tissue after it is scraped off from the walls of the body lumens. The reservoirs 190 extend from the outer surface 161 to the inner surface 162, as shown in Figures 1 and 2. As an alternative to having reservoirs 190 trap the collected tissue, a lumen (not shown) may extend the longitudinal length of the curette 100 and be used to collect the scraped tissue cells.

Scraping element 160 may be formed from any suitable metallic alloy, including stainless steel and nitinol. Additionally, scraper element 160 may be formed from any suitable biocompatible polymer known to one of ordinary skill in the art.

The scraping element 160 may be detachable from the base 166 of tubular shaft 130. For example, it can be removed and taken for laboratory analysis following a tissue sampling procedure. A new scraping element can then be affixed to the tubular shaft 130 for a subsequent procedure. The tubular shaft 130 is preferably a reusable component.

Figure 2 is a side view of the wire-guided curette 100 shown in Figure 1. Figure 2 shows that there is a gap between the aperture 170 and the distal tip 165 of the scraping element 160. This gap is sufficient for the wire guide 110 to exit through aperture 170 without impeding the scraping element 160 during the curettage procedure. Reservoirs 190 are shown extending from the outer surface 161 to the inner surface 162.

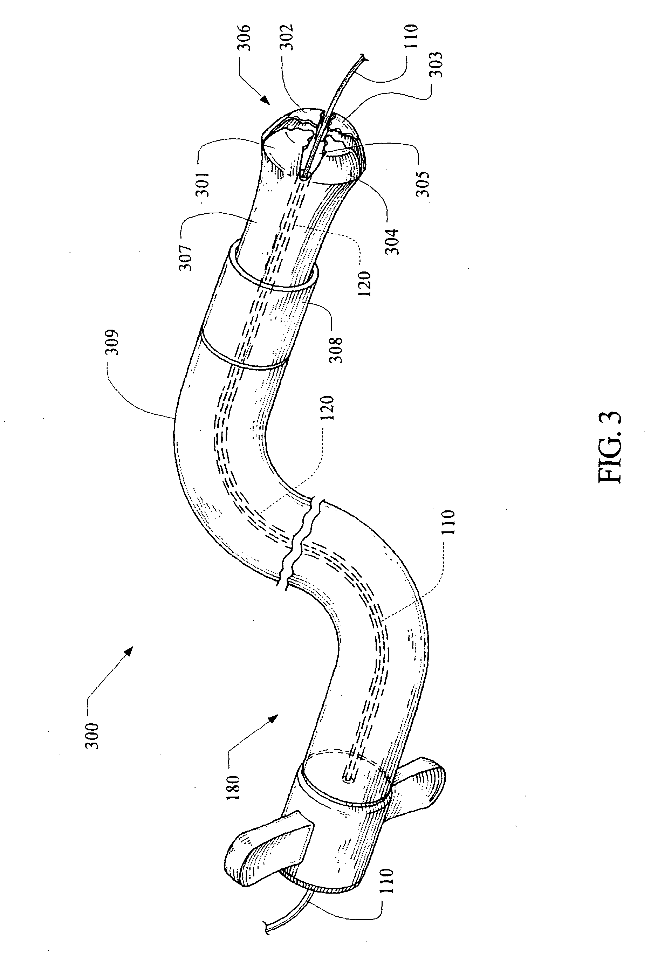

Although the scraping element 160 of the curette 100 of Figure 1 may have any surface to scrape tissue, some curette procedures may require the scraping process to be relatively less traumatic. As an example, Figure 3 shows a curette 300 having four atraumatic scraping surfaces 301-304. Each of the surfaces 301-304 is curved inwardly toward a longitudinal axis of the curette 100 to create an atraumatic distal end of the wire-guided curette 300. The scraping element 306 contains a stem 307, as shown in Figure 3. The stem 307 is affixed to base 308 of tubular shaft 309. The scraping element 306 and the stem 307 are a single structure which can be detached from the base 308 following the curettage procedure for sampling and/or removing tissue. The four scraping surfaces 301-304 extend circumferentially about the shaft 309 to enable the physician to obtain tissue from one or more target locations along the body lumen without the need for aligning a particular outer scraping surface with the target tissue. In other words, at least one of the scraping surfaces 301-304 of the scraping element 306 may always be aligned with and capable of removing the target tissue regardless of the orientation of the scraping element 306 within the body lumen. Therefore, potential trauma to the patient and curettage procedure time may be minimized. Although scraping element 306 has been shown as having four individual scraping surfaces 301-304, it may comprise a single scraping structure that circumferentially extends about the entire perimeter of the scraping element 306.

Similar to the embodiments of Figures 1 and 2, the curette 300 of Figure 3 is shown having a wire guide lumen 120 extending longitudinally to the proximal portion 180. Other variations of the wire guide lumen 120 are contemplated. For example, the curette 300 may have an abbreviated wire guide lumen 120. (See Figure 6). Although the surfaces 301-304 are curved, they maintain a sufficient opening 305 for the wire guide 110 to pass therethrough as the wire guide 110 emerges distally from the wire guide lumen 120.

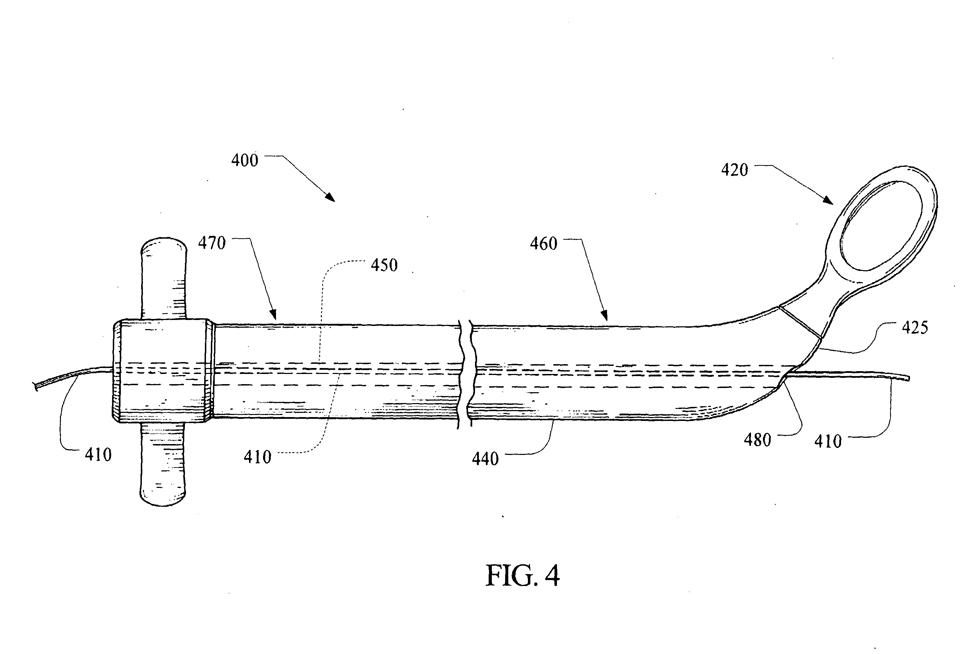

Although a spoon-shaped scraping element has been shown in Figures 1 and 2, other shapes of the scraping element are contemplated. For example, having a scraping element that is curvilinear and angled with respect to the shaft of the curette may be advantageous for particular body lumens having a tortuous geometry. As an example, Figure 4 shows a wire-guided curette 400 having an angled loop-shaped scraping element 420. The angled loop-shaped scraping element 420 is affixed to stem 425, and the stem 425 is affixed to the shaft 440. Figure 4 shows the shaft 440, stem 425, and angled loop-shaped scraping element 420 as a unitary structure. Alternatively, the scraping element 420 may be an individual component that is detachable from the stem 425. After use, the scraping element 420 can be removed and taken for laboratory analysis. A new scraping element 420 can then be affixed to the shaft 440. The stem 425 and loop-shaped scraping element 420 are shown angled away from the longitudinal axis of the shaft 440 a sufficient amount. This enables the wire guide 410 to exit opening 480 without interfering with any surface of the loop-shaped scraping element 420 during the curettage procedure. The wire guide 410 is disposed within wire guide lumen 450, which is shown to extend from the distal portion 460 to the proximal portion 470 of shaft 440. The wire guide lumen 450 is substantially parallel to the longitudinal axis of the shaft 440. Other variations of wire guide lumen 450 are contemplated. For example, the curette 400 may have an abbreviated wire guide lumen 450 to facilitate short wire guide exchange of the wire guide 410 and device 400. (See Figure 6).

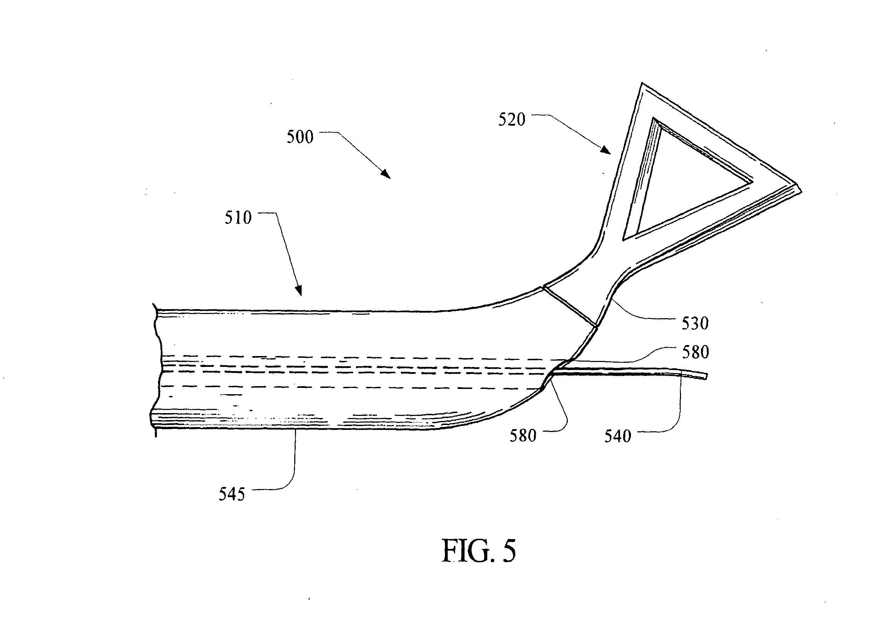

In addition to angled, curvilinear-shaped scraping elements, other shapes are contemplated. For example, Figure 5 show shows the distal portion 510 of a wire-guided curette 500 having a triangular-shaped scraping element 520. The triangular-shaped scraping element 520 is affixed to stem 530. As with the curette 400 of Figure 4, the triangular-shaped scraping element 520 does not interfere with loading of the wire guide 540 through wire guide lumen 550. The curette 500 may have an abbreviated wire guide lumen 550 to facilitate short wire guide exchange of the wire guide 540 and device 500.

Having described the structural elements of the wire-guided curette, an example of a method of its use within the gastrointestinal tract will now be described, with reference to Figure 1.

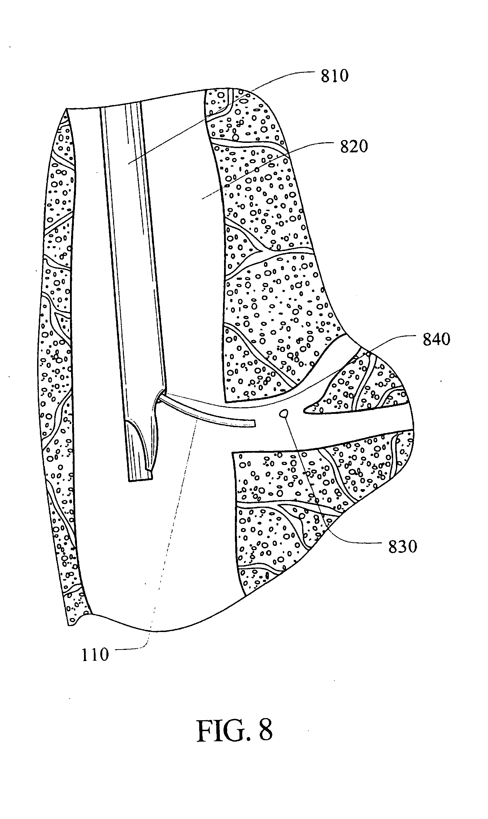

A physician advances an endoscope 810 within a desired region of the gastrointestinal lumen 820 where the target tissue 830 to be removed is located (Figure 8). If possible, the distal end of the endoscope 810 is advanced in close proximity to the target tissue 830. With the endoscope 810 deployed in a desired position, the wire guide 110 is loaded through the accessory channel of the endoscope 810 until it emerges from the distal end of the channel and enters into the target site. After the surgeon has positioned wire guide 110 in close proximity to the target site, the wire-guided curette 100 of Figure 1 can be loaded into the accessory channel 840. In particular, the wire guide lumen 120 receives the wire guide 110, which serves as a stable guide to facilitate deployment of the wire-guided curette 100. The physician may maneuver control handle 150 to aid in manipulating the scraper element 160 towards the target site.

After the scraper element 160 has reached the target site, the curettage procedure may begin. The physician applies a suitable amount of pressure at the control handle 150 to cause the scraper element 160 to contact and scrape away cells from the tissue 830 at the target site. The scraper element 160 may engage the target tissue 830 via back and forth movements to scrape away cells for subsequent sampling. Alternatively or in addition, the physician may apply torque to the shaft 130 to rotate the scraper element 160 against the tissue 830. Any surface or combination of the inner surface 162, outer surface 161, and edge 163 of the scraping element 160 may be used to engage and scrape away cells from the target tissue 830. Reservoirs 190 may help to trap the tissue 830 after it is scraped off from the body lumen.

After a sufficient amount of cells have been scraped, the curette 100 may be withdrawn from the patient. Upon withdrawal the scraper element 160 may be detached from the base 166. The scraped cells are removed from the reservoirs 190 in order for subsequent sampling of the cells to be conducted.

The curette 100 may also be used to sweep away strictures within a body lumen. The spoon-shape scraper element 160 of Figure 1 enables a scoop-like movement that may be used to sweep away the strictures that are impeding the body lumen. Additionally, the angled curettes 400 and 500 shown in Figures 4 and 5 may be effective in body lumens that are too tortuous for the curettes of Figure 1.

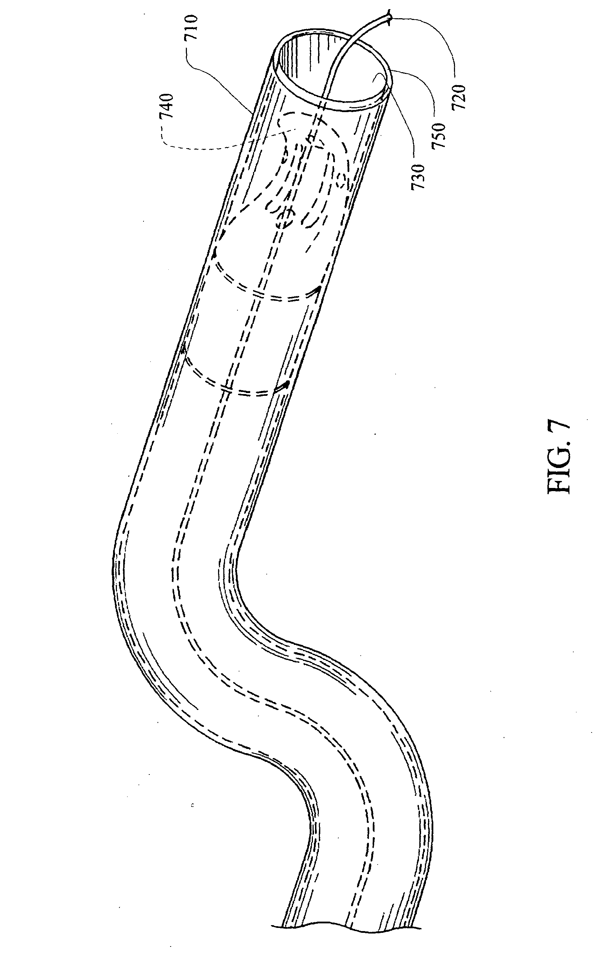

Alternatively, when loading the wire-guided curette through the accessory channel of the endoscope, the wire-guided device may have a protective sheath disposed over the device. Figure 7 shows a protective sheath 710 disposed over a wire-guided curette 730. A wire guide 720 extends through the lumen of the curette 730. The protective sheath 710 may help to prevent the scraper element 740 from damaging the accessory channel of the endoscope that the curette 730 is loaded through. As shown in Figure 7, the protective sheath 710 may be a tubular structure which is open on the proximal end (not shown) and distal end 750. After the curette 730 reaches the target tissue site, the sheath 710 may be retracted to expose the scraping element 740 for performing the curettage procedure. Alternatively, the wire-guided curette 730 may be extended pass the distal end 750 of the protective sheath 710 to expose the scraping element 740. After the curettage procedure is completed, the scraping element 740 may be retracted into the protective sheath 710 and subsequently withdrawn from the target tissue site through the accessory channel of the endoscope.

The wire-guided curette may be used without an endoscope and in other areas besides the gastrointestinal lumen. As can be seen, unlike conventional curettes, the above-described curettes are capable of being wire guided to remote body cavities where curettage procedures may be conducted.

The above figures and disclosure are intended to be illustrative and not exhaustive. This description will suggest many variations and alternatives to one of ordinary skill in the art. All such variations and alternatives are intended to be encompassed within the scope of the attached claims. Those familiar with the art may recognize other equivalents to the specific embodiments described herein which equivalents are also intended to be encompassed by the attached claims.

## Claims

1. A wire-guided curette device, comprising: a tubular shaft (130) extending between a proximal portion (180) and a distal portion (140), the distal portion (140) adapted to be introduced into the body lumen, the proximal portion(180) adapted to remain outside the lumen so as to allow manipulation of the distal portion;
a scraping element (160) operably connected to the distal portion (140) of the tubular shaft, the scraping element (160) having at least one surface configured to remove tissue from the body lumen;
a wire guide lumen (120) extending longitudinally through at least the distal portion of the tubular shaft, the wire guide lumen (120) comprising an aperture (170) disposed near the scraping element, the aperture (170) being spaced apart from the scraping element, wherein the wire guide lumen is adapted to receive a wire guide (110); and
wherein the scraping element (160) comprises one or more reservoirs (190) for collecting removed tissue, wherein the one or more reservoirs (190) are separate from the wire guide aperture (170).

2. The device of claim 1, wherein the scraping element is detachable from the distal portion of the shaft.

3. The device of claim 1, wherein a wire guide extends through the wire guide lumen and exits through the aperture, the aperture being located sufficiently proximal to a distal end of the scraping element so that the wire guide does not interfere with a curettage procedure.

4. The device of claim 1, wherein the scraping element comprises a plurality of scraping surfaces, each of the plurality of scraping surfaces configured to contact the body lumen to surgically scrape tissue in a curettage procedure.

5. The device of claim 1, wherein the scraping element is a curved surface, the curved surface circumscribing the distal portion of the shaft, the aperture being disposed in a central portion of the curved surface.

6. The device of claim 1, the tubular shaft having a length of at least about 100 centimeters.

7. The device of claim 1, wherein the scraping element is spoon-shaped.

8. The device of claim 1, the distal portion further comprising a short wire guide port adapted to receive the wire guide.

## Patentansprüche

1. Drahtgeführte Kürettenvorrichtung, die Folgendes umfasst: einen röhrenförmigen Schaft (130), der sich zwischen einem proximalen Abschnitt (180) und einem distalen Abschnitt (140) erstreckt, wobei der distale Abschnitt (140) dazu ausgelegt ist, in das Körperlumen eingeführt zu werden, wobei der proximale Abschnitt (180) dazu ausgelegt ist, außerhalb des Lumens zu bleiben, um die Manipulation des distalen Abschnitts zu ermöglichen; ein Schabeelement (160), das mit dem distalen Abschnitt (140) des röhrenförmigen Schafts in Wirkverbindung steht, wobei das Schabeelement (160) zumindest eine Fläche aufweist, die konfiguriert ist, Gewebe aus dem Körperlumen zu entfernen; ein Drahtführungslumen (120), das sich in Längsrichtung durch zumindest den distalen Abschnitt des röhrenförmigen Schafts erstreckt, wobei das Drahtführungslumen (120) eine Öffnung (170) umfasst, die in der Nähe des Schabeelements angeordnet ist, wobei die Öffnung (170) vom Schabeelement beabstandet ist, worin das Drahtführungslumen dazu ausgelegt ist, eine Drahtführung (110) aufzunehmen; und worin das Schabeelement (160) ein oder mehr Reservoirs (190) zur Aufnahme von entferntem Gewebe umfasst, worin das eine oder die mehreren Reservoirs (190) von der Drahtführungsöffnung (170) getrennt sind.

2. Vorrichtung nach Anspruch 1, worin das Schabeelement vom distalen Abschnitt des Schafts gelöst werden kann.

3. Vorrichtung nach Anspruch 1, worin eine Drahtführung sich durch das Drahtführungslumen erstreckt und durch die Öffnung austritt, wobei die Öffnung ausreichend proximal zu einem distalen Ende des Schabeelements angeordnet ist, damit die Drahtführung bei einem Kürettageeingriff nicht stört.

4. Vorrichtung nach Anspruch 1, worin das Schabeelement eine Vielzahl von Schabeflächen umfasst, wobei jede der Vielzahl von Schabeflächen konfiguriert ist, mit dem Körperlumen in Berührung zu stehen, um Gewebe bei einem Kürettageeingriff chirurgisch abzuschaben.

5. Vorrichtung nach Anspruch 1, worin das Schabeelement eine gekrümmte Fläche ist, wobei die gekrümmte Fläche den distalen Abschnitt des Schafts abgrenzt, wobei die Öffnung in einem mittleren Abschnitt der gekrümmten Fläche angeordnet ist.

6. Vorrichtung nach Anspruch 1, wobei der röhrenförmige Schaft eine Länge von zumindest ungefähr 100 Zentimeter aufweist.

7. Vorrichtung nach Anspruch 1, worin das Schabeelement löffelförmig ist.

8. Vorrichtung nach Anspruch 1, wobei der distale Abschnitt ferner eine kurze Drahtführungsöffnung, die zur Aufnahme der Drahtführung ausgelegt ist, umfasst.

## Revendications

1. Dispositif de curette filoguidée, comportant : une tige (130) tubulaire s'étendant entre une portion (180) proximale et une portion (140) distale, la portion (140) distale étant conçue pour être introduite dans la lumière corporelle, la portion (180) proximale étant conçue pour rester à l'extérieur de la lumière de façon à permettre la manipulation de la portion distale ;
un élément (160) de raclage relié de manière fonctionnelle à la portion (140) distale de la tige tubulaire, l'élément (160) de raclage ayant au moins une surface configurée pour prélever du tissu de la lumière corporelle ;
une lumière (120) de fil de guidage traversant longitudinalement au moins la portion distale de la tige tubulaire, la lumière (120) de fil de guidage comportant une ouverture (170) placée près de l'élément de raclage, l'ouverture (170) étant espacée de l'élément de raclage, dans lequel la lumière de fil de guidage est conçue pour recevoir un fil de guidage (110) ; et
dans lequel l'élément (160) de raclage comporte un ou plusieurs réservoirs (190) destinés à recueillir le tissu prélevé, dans lequel ledit un ou lesdits plusieurs réservoirs (190) sont séparés de l'ouverture (170) du fil de guidage.

2. Dispositif selon la revendication 1, dans lequel l'élément de raclage est détachable de la portion distale de la tige.

3. Dispositif selon la revendication 1, dans lequel un fil de guidage traverse la lumière de fil de guidage et ressort par l'ouverture, l'ouverture étant située à une distance suffisamment proximale d'une extrémité distale de l'élément de raclage de sorte que le fil de guidage ne gêne pas une procédure de raclage.

4. Dispositif selon la revendication 1, dans lequel l'élément de raclage comporte plusieurs surfaces de raclage, chacune desdites surfaces de raclage étant configurée pour venir en contact avec la lumière corporelle pour racler chirurgicalement le tissu lors d'une procédure de raclage.

5. Dispositif selon la revendication 1, dans lequel l'élément de raclage est une surface courbe, la surface courbe entourant la portion distale de la tige, l'ouverture étant placée dans une portion centrale de la surface courbe.

6. Dispositif selon la revendication 1, la tige tubulaire étant d'une longueur d'au moins environ 100 cm.

7. Dispositif selon la revendication 1, dans lequel l'élément de raclage a la forme d'une cuillère.

8. Dispositif selon la revendication 1, la portion distale comportant en outre un orifice pour un fil de guidage court conçu pour recevoir le fil de guidage.
